(19) **Europäisches Patentamt**

**European Patent Office**

**Office européen des brevets**

(11) **EP 1 010 982 B1**

(12) **EUROPÄISCHE PATENTSCHRIFT**

(45) Veröffentlichungstag und Bekanntmachung des Hinweises auf die Patenterteilung:
**15.02.2006 Patentblatt 2006/07**

(51) Int Cl.:
***G01N 33/86*** *(2006.01)*

(21) Anmeldenummer: **99123057.4**

(22) Anmeldetag: **20.11.1999**

(54) **Verfahren zum Nachweis von Defekten eines mehrstufigen, multifaktoriellen biochemischen Reaktionsystems**

Method for detecting defects in a multisteps and multifactorial biochemical system

Méthode de localisation de défauts dans un système biochimique multifactorielle et multiétapes

(84) Benannte Vertragsstaaten:
**AT BE CH CY DE DK ES FI FR GB GR IE IT LI LU MC NL PT SE**

(30) Priorität: **17.12.1998 DE 19858278**

(43) Veröffentlichungstag der Anmeldung:
**21.06.2000 Patentblatt 2000/25**

(73) Patentinhaber: **Dade Behring Marburg GmbH 35001 Marburg (DE)**

(72) Erfinder:
• **Siegemund, Annelie, Dr. 04457 Mölkau (DE)**
• **Siegemund, Thomas 04457 Mölkau (DE)**

(56) Entgegenhaltungen:
WO-A-91/01383    WO-A-95/12127
US-A- 5 197 017

• **BERTINA R M; VAN DER MAREL-VAN NIEUWKOOP W; LOELIGER E A: "SPECTROPHOTOMETRIC ASSAYS OF PROTHROMBIN IN PLASMA OF PATIENTS USING ORAL ANTICOAGULANTS" THROMBOSIS AND HAEMOSTASIS, Bd. 42, 1979, Seiten 1296-1305, XP000893035**
• **CORRIGAN J J JR; EARNEST D L: "FACTOR-II ANTIGEN IN LIVER DISEASE AND WARFARIN INDUCED VITAMIN K DEFICIENCY CORRELATION WITH COAGULANT ACTIVITY USING ECHIS VENOM" AMERICAN JOURNAL OF HEMATOLOGY, Bd. 8, 1980, Seiten 249-255, XP000893099**
• **ROSENDAAL FR, SISCOVICK DS, SCHWARTZ SM, PSATY BM, RAGHUNATHAN TE, VOS HL: "A common prothrombin variant (20210 G to A) increases the risk of myocardial infarction in young women" BLOOD, Bd. 90, Nr. 5, 1. September 1997 (1997-09-01), Seiten 1747-1750, XP000892937**
• **BROWN K, LUDDINGTON R, WILLIAMSON D, BAKER P, BAGLIN T: "Risk of venous thromboembolism associated with a G to A transition at position 20210 in the 3'-untranslated region of the prothrombin gene" BRITISH JOURNAL OF HAEMATOLOGY, Bd. 98, Nr. 4, September 1997 (1997-09), Seiten 907-909, XP000892928**

EP 1 010 982 B1

## Beschreibung

Die vorliegende Erfindung betrifft ein Verfahren zur Identifikation von Defekten im Gerinnungs-, Fibrinolyse- und Komplementsystem.

Thrombosen und Blutung zählen heute, vor allem in den Industrieländern, zu den häufigsten Ursachen für Krankheit und Tod. Am bekanntesten sind Herzinfarkt, Schlaganfall und Lungenembolie. Bei überstandener Thromboembolie oder Blutung ist die Vitalität des Patienten in den meisten Fällen eingeschränkt, auf der einen Seite stehen Folgeerscheinungen wie Lähmungen, postthrombotisches Syndrom oder Organschäden, auf der anderen Seite aufwands- und kostenintensive Anschlußbehandlungen, wie Kuren, Physiotherapie und Medikation zur Verbesserung der gesundheitlichen Situation und der Prävention weiterer Komplikationen.

Vor allem in der Erforschung der Thromboseursachen sind in den letzten Jahren große Fortschritte gemacht worden, dazu zählen die Entdeckung der APC-Resistenz, der Lupus Antikoagulantien, der Hyperhomocysteinämie und der Prothrombinvariante G20210A. Dennoch ist bei ca. 50 % aller Fälle keine Ursache erkennbar. Die wichtigsten hämostaseologischen Blutungsursachen sind bekannt, dazu gehören die verschiedenen Formen der Hämophilie, das von Willebrand-Jürgens-Syndrom und seltenere Mutationen der einzelnen Gerinnungsfaktoren.

Liegen solche Defekte vor, kommt es zur Störung des hämostatischen Gleichgewichtes, das Verhältnis zwischen pro- und antikoagulatorischen Faktoren ist zugunsten einer Seite verschoben. Zu Thrombosen führen meist Defekte, die eine erhöhte Ausschüttung an prokoagulatorischen Faktoren zur Folge haben, so zum Beispiel die Prothrombinvariante G20210A, mangelnde Freisetzung von Inhibitoren, wie bei Protein C-, Protein S- oder AT III-Mangel oder die Verhinderung der Inhibierung durch veränderte Rezeptoren, bekannteste Form die Resistenz gegenüber der aktiven Form des Protein C (APC-Resistenz: Bertina RM, Clin Chem; 43(9): 1678-83). Dazu kommen Defekte im Fibrinolysesystem, die den Abbau entstandener Gerinnsel reduzieren.

Aufgabe des Labors ist es, solche Defekte zu identifizieren, damit der behandelnde Arzt das individuelle Risiko des Patienten abschätzen und darauf reagieren kann. Dabei kommen verschiedene Verfahren der Diagnosestellung zum Einsatz: Globalteste erfassen das Zusammenspiel mehrerer Komponenten des Gerinnungssystems. Die Prothrombinzeit (PT) erfaßt den Zustand des exogenen, die Partielle Thromboplastinzeit (aPTT) den Zustand des endogenen Gerinnungssystems. Weiterhin kommen modifizierte Globalteste bei Analyse des Protein C-Systems und bei der Diagnose von Lupus Antikoagulantien zum Einsatz. Dem gegenüber stehen Einzelteste, die jeweils nur eine einzelne Komponente erfassen und eine sowohl qualitative als auch quantitative Analyse erlauben. Bei der Fragestellung nach erworbenen Hemmkörpern kommen modifizierte Einzelteste zum Einsatz. Für die Überwachung des Gerinnungsstatus antikoagulierter Patienten wurde der neue Parameter XAPC/ECAR, der Quotient aus den Einzeltest-Meßwerten XAPC und ECAR, als diagnostischer Marker alternativ zur Prothrombinzeit überprüft [Bertina, R. M. et al. (1979) Thrombos. Haemostas. 42, 1296-1305). J. J. Corrigan & D. L. Eamest (1980) Am. J. Hematol. 8, 249-255, offenbaren zwei spezifische Einzelteste zur Differenzierung von Faktor II-Defekten in Synthesedefekte bzw. Modifikationsdefekte bei Patienten, die eine verlängerte Prothrombinzeit aufweisen. In der Patentanmeldung WO-A-91/01383 wird eine Methode zur Identifikation von Defekten eines Einzelfaktors offenbart, die darauf beruht, daß die Meßwerte einer Patientenprobe mit den parallel bestimmten Meßwerten eines Standardnormalplasmas und mit Referenzmeßwerten aus einer Datenbank verglichen werden. Trotz der vielfältigen Methoden, die zur Auswahl stehen, ist eine genaue Diagnose oft nicht möglich: Die Grenzen zwischen "positivem" und "negativem" Befund sind nicht deutlich oder Medikamente, wie z.B. Antikoagulantien, beeinflussen das Testergebnis nachhaltig.

Eindeutige Diagnosen bei angeborenen Defekten liefert die DNS-Analyse. Diese eignet sich vor allem bei Defekten, die auf einer einzelnen Mutation beruhen, wie Faktor V-Leiden (eine Form der APC-Resistenz) oder die Prothrombinvariante G20210A. Liegen heterogene Defekte vor, ist auch die Genanalyse äußerst erschwert. Doch treten im Zusammenhang mit der DNS-Analyse andere Probleme auf. Nur wenige Laboratorien sind in der Lage eine solche Untersuchung durchzuführen, dazu kommen die hohen Kosten, die mit ihr verbunden sind.

Ein Ziel ist es also, vor der DNS-Analyse das Patientenkollektiv zu selektieren, um Arbeit und Kosten zu sparen. Bekanntestes Beispiel ist hier die Resistenz gegen Protein C. Es gibt verschiedene Teste zur Untersuchung auf APC-Resistenz. Erhält man einen positiven Befund, kann man eine Genanalyse durchführen, ob die sehr häufige Form des Faktor V-Leidens vorliegt. Eine solche Trennung zwischen "positiv" und "negativ" hat noch einen zweiten entscheidenden Vorteil. Soll nach einem unbekannten Defekt gesucht werden, könnten Patientenkollektive getrennt und "potentiell positive" herausselektiert werden und per Gen-Sequenzierung analysiert werden. Bisher wurden diese Kollektive nach der Anamnese selektiert.

Ein sehr gutes Beispiel für diese Probleme ist die Prothromvariante G20210A. Diese Punktmutation ist mit erhöhten Prothrombinspiegeln verbunden, welche zu einem erhöhten Thromboserisiko führen (Poortn, Blood 1996; 88(10): 3698-703). Publikationen weisen auf ein erhöhtes Risiko für Herzinfarkte (Rosendahl, Blood 1997; 90(5)1747-50) und venöse Thrombosen (Brown, Br.J. Haematol; 98(4):907-9) hin. Es konnte aber auch nachgewiesen werden, daß eine Diskriminierung zwischen Mutationsträger und Wildtyp mit Hilfe des Prothrombinspiegels nicht möglich ist, da beide Gruppen nicht trennbar sind (Poortn, Blood 1996; 88(10): 3698-703; siehe auch eigene Ergebnisse). Patienten, die unter oraler Antikoagulation standen, mußten ausgeschlossen werden.

Wir fanden überraschenderweise eine Möglichkeit, zwischen Trägern der Mutation und Trägern des Wildtyp-Gens eine sehr gute Trennung zu erzielen. Dies konnte dadurch erreicht werden, daß wir das Ergebnis eines Einzeltestes in Bezug zu einem dazugehörigen Suchtest gesetzt haben. Die zusammengehörigen Tests sind an sich dem Fachmann bekannt. Der Bezug, in den Einzeltest und Suchtest gesetzt werden können, ist die Verhäfthisbildung (Ratio), die Ermittlung des Produktes, der Differenz oder der Summe. Für die Diskriminierung wichtige Größen sind die Sensitivität, Spezifität und der Cut-Off. Wie aus den Beispielen hervorgeht, kann durch Variation des Cut-Off die Spezifität auf Kosten der Sensitivität erhöht werden. Bevorzugterweise wird eine geringere Sensitivität angenommen, um die Spezifität zu erhöhen. Zur Erhöhung der Spezifität bevorzugterweise wird eine Sensitivität von maximal 100 %, besonders bevorzugterweise eine Sensitivität von 95 - 100 %, ganz besonders bevorzugterweise eine Spezifität von 80- 95 % angenommen. Aufgrund der technischen Lehre der vorliegenden Erfindung kann der Fachmann durch einfache Versuche das gewünschte Verhältnis von Sensitivität und Spezifität durch die Bestimmung eine geeigneten Cut-off Wertes festlegen.

So lassen sich auch erworbenen Erkrankungen diagnostizieren, da diese ebenfalls hypo- oder hyperkoagulatorische Zustände hervorrufen, die sich nur durch leichte, für sich genommen oft nicht signifikante Veränderung der Aktivität des betroffenen Parameters auszeichnen.

Das Verfahren eignet sich besonders zum Nachweis von Defekten eines Analyten aus der Gruppe Faktor II, V, VII, X, XI und XII.

**Abbildungen:**

Abbildung 1     zeigt die Bestimmung von Mutationsträgern nach dem Verfahren gemäß dem Stand der Technik (Bestimmung der Faktor II-Konzentration) an 46 Blutspendern (Wildtyp) und 54 Thrombosepatienten.

Abbildung 2     zeigt die erfindungsgemäße Bestimmung von Mutationsträgern am gleichen Probenkollektiv (Ratioverfahren) nach folgender Formel :

$$\text{Ratio} = \text{Faktor II-Konzentration [\% d.N.]/Prothrombinzeit (PT) [\% d.N.]}$$

Abbildung 3     zeigt die erfindungsgemäße Bestimmung von Mutationsträgern am gleichen Probenkollektiv.

Die folgenden Beispiele sollen die Erfindung erläutern, jedoch die Ansprüche in keiner Weise einschränken.

Beispiel 1

Als Beispiel soll die Prothrombinvariante G20210A aufgeführt werden.

Die hier vorliegenden Daten wurden wie folgt ermittelt: 46 Blutspender und 53 Thrombose-Patienten wurden mit Hilfe einer PCR aus EDTA-Blut (DNS-Isolierung und Amplifikation, alleispezifische Hybridisierung mit markierten Sonden in Mikrotiterstreifen; Medizinische Diagnostische Produkt GmbH) in die Gruppen Mutationsträger (40) und Wildtypen (59) geteilt. Aus Citratblut wurden für beide Gruppen die Faktor II-Konzentration (Faktor II-Testkit, Dade Behring Marburg GmbH; ACL, Instrumentation Laboratory) und die Prothrombinzeit (Immunoplastin IS, Immuno GmbH, BCT Dade Behring Marburg GmbH) bestimmt.

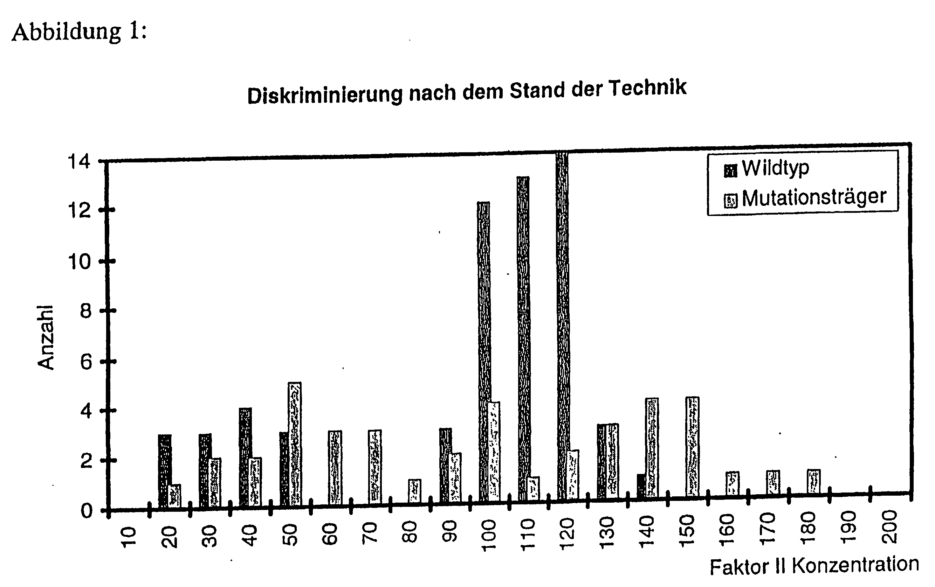

Die Verteilung der Faktor II-Konzentration ist in Abbildung 1 dargestellt (Verfahren nach dem Stand der Technik).

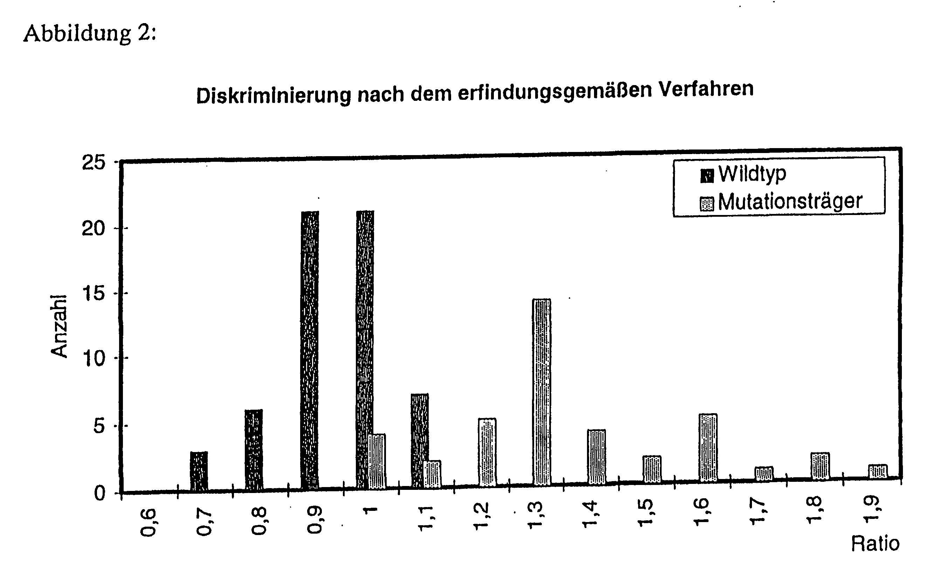

In Abbildung 2 ist die Verteilung der Ratio nach folgender Formel dargestellt (erfindungsgemäßes Verfahren) :

$$\text{Ratio} = \text{Faktor II-Konzentration [\% d.N.]/Prothrombinzeit (PT) [\% d.N.]}$$

Um die Vorzüge des erfindungsgemäßen Verfahrens zu demonstrieren, folgt eine statistische Auswertung beider Verfahren. Im ersten Schritt werden Sensitivitätswerte von 100%, 95% und 90% vorausgesetzt und für beide Verfahrensweisen jeweils Cut-Off und Spezifität ermittelt:

|  | Diskriminierung nach | |
|---|---|---|
|  | Faktor II-Konzentration (Stand der Technik) | Ratio (erfindungsgem. Verf.) |
| Sensitivität | 100% | 100 % |
| Cut-Off | 13% d.N. | 0,95 |
| Spezifität | 0% | 64% |
| Sensitivität | 95% | 95% |
| Cut-Off | 29% d.N. | 0,99 |
| Spezifität | 10% | 83% |
| Sensitivität | 90% | 90% |
| Cut-Off | 33% d.N. | 1,02 |
| Spezifität | 10% | 92% |

Im zweiten Schritt wird eine Spezifität von 100% angenommen:

|  | Diskriminierung nach | |
|---|---|---|
|  | Faktor II-Konzentration (Stand der Technik) | Ratio (erfindungem. Verfahren) |
| Spezifität | 100% | 100% |
| Cut-Off | 132% d.N. | 1,07 |
| Sensitivität | 28% | 85% |

Aus den erhaltenen Werten gehen die Vorzüge des erfindungsgemäßen Verfahrens deutlich hervor.

Beispiel 2

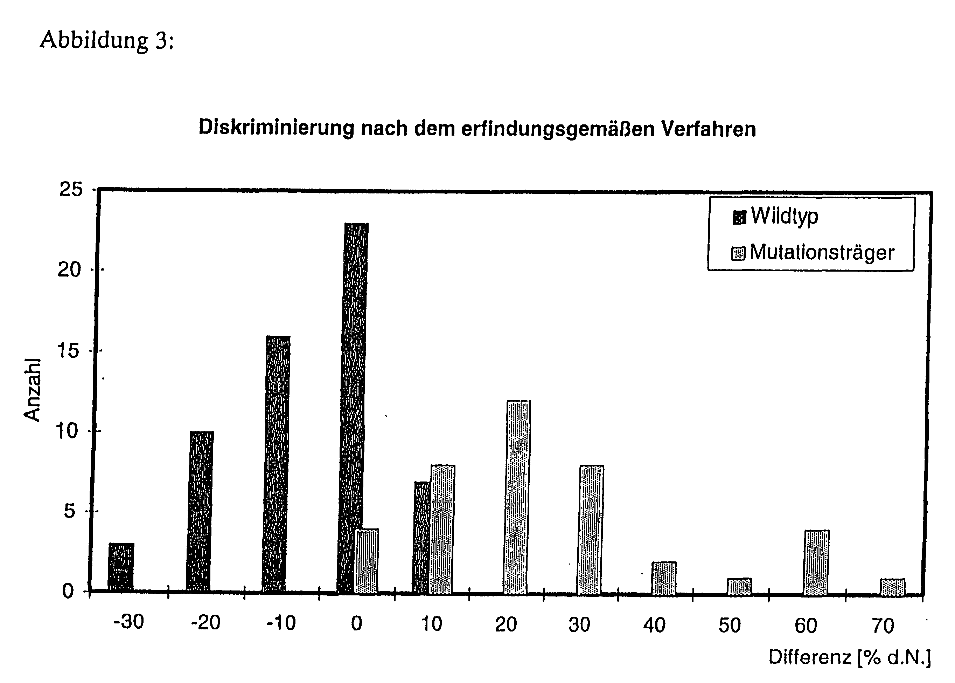

Es gelten die gleichen Voraussetzungen wie bei Beispiel 1. In Abbildung 3 ist die Verteilung nach Differenzenbildung dargestellt:

$$\text{Differenz [\% d.N.]} = \text{Prothrombinzeit (PT) [\% d.N.]} - \text{Faktor II-Konzentration [\% d.N.]}$$

Um die Vorzüge des erfindungsgemäßen Verfahrens zu demonstrieren, folgt eine statistische Auswertung beider Verfahren. Im ersten Schritt werden Sensitivitätswerte von 100 %, 95% und 90% vorausgesetzt und für beide Verfahrensweisen jeweils Cut-Off und Spezifität ermittelt:

| | Diskriminierung nach | |
|---|---|---|
| | Faktor II-Konzentration (Stand der Technik) | Differenz (erfindungsgem. Verf.) |
| Sensitivität | 100% | 100% |
| Cut-Off | 13%d.N. | -6%d.N. |
| Spezifität | 0% | 63% |
| Sensitivität | 95% | 95% |
| Cut-Off | 29% d.N. | -1% d.N. |
| Spezifität | 10% | 80% |
| Sensitivität | 90% | 90% |
| Cut-Off | 33% d.N. | 2% d.N. |
| Spezifität | 10% | 93% |

Im zweiten Schritt wird eine Spezifität von 100% angenommen:

| | Diskriminierung nach | |
|---|---|---|
| | Faktor II-Konzentration (Stand der Technik) | Differenz (erfindungsgem. Verf.) |
| Spezifität | 100% | 100% |
| Cut-Off | 132%d.N. | -6%d.N. |
| Sensitivität | 28% | 78% |

Aus den erhaltenen Werten gehen die Vorzüge des erfindungsgemäßen Verfahrens deutlich hervor.

**Patentansprüche**

1. Verfahren zum Nachweis von Defekten eines ausgewählten Einzelfaktors (Analyt) eines mehrstufigen, multifaktoriellen biochemischen Reaktionssystems aus der Gruppe Fibrinolysesystem, Gerinnungssystem und Komplementsystem, das folgende Schritte einschließt:

   a) Bestimmung des Analyten,
   b) Bestimmung eines Teilbereiches des Reaktionssystems, der den in Schritt a) bestimmten Analyten einschließt,
   c) Ermittlung der Ratio, des Produktes, der Differenz oder der Summe der unter a) und b) erhaltenen Werte und
   d) Diskriminierung zwischen "Defektträgern" und "Normalpopulation", dadurch dass ein geeigneter "Cut-Off" festgelegt wird.

2. Verfahren gemäß Anspruch 1), wobei der Analyt ausgewählt ist aus der Gruppe:
   Faktor II, V, VII, X, XI und XII.

**3.** Verfahren gemäß einem der Ansprüche 1) bis 2), wobei der unter b) durchzuführende Globaltest ausgewählt ist aus der Gruppe:
Prothrombinzeit und partielle Prothrombinzeit.

**Claims**

**1.** Method for detection of defects of a selected individual factor (analytical parameter) of a multistage, multifactorial biochemical reaction system from the group consisting of fibrinolysis system, clotting system and complement system, which includes the following steps:

a) determination of the analytical parameter,
b) determination of a part range of the reaction system which includes the analytical parameter determined in step a),
c) determination of the ratio, the product, the difference or the total of the values obtained under a) and b), and
d) discrimination between "different carriers" and "normal population" by specifying a suitable "cut-off".

**2.** Method according to Claim 1, wherein the analytical parameter is chosen from the group consisting of:
factor II, V, VII, X, XI and XII.

**3.** Method according to one of Claims 1) to 2), wherein the global test to be carried out under b) is chosen from the group consisting of:
prothrombin time and partial prothrombin time.

**Revendications**

**1.** Procédé pour la détection d'anomalies d'un facteur individuel (analyte) d'un système réactionnel biochimique multifactoriel à plusieurs stades, choisi parmi le système de fibrinolyse, le système de coagulation et le système du complément, qui comprend les étapes suivantes :

a) détermination de l'analyte,
b) détermination d'une zone partielle du système réactionnel qui inclut l'analyte déterminé dans l'étape a),
c) détermination du rapport, du produit, de la différence ou de la somme des valeurs obtenues en a) et b) et
d) distinction entre "porteurs d'anomalie" et "population normale", par établissement d'un "seuil" approprié.

**2.** Procédé selon la revendication 1, dans lequel l'analyte est choisi dans le groupe : facteur II, V, VII, X, XI et XII.

**3.** Procédé selon la revendication 1 ou 2, dans lequel le test global à effectuer en b) est choisi parmi le temps de prothrombine et le temps partiel de prothrombine.

Abbildung 1:

**Diskriminierung nach dem Stand der Technik**

EP 1 010 982 B1

Abbildung 2:

Diskriminierung nach dem erfindungsgemäßen Verfahren

Abbildung 3:

**Diskriminierung nach dem erfindungsgemäßen Verfahren**

EP 1 010 982 B1